# FASCICULE DE BREVET EUROPEEN

(11) **EP 0 826 029 B1**
(45) Date de publication et mention de la délivrance du brevet: **31.10.2001**
(21) Numéro de dépôt: 96915079.6
(22) Date de dépôt: 23.04.1996
(51) Int. Cl.: C12C 1/00, C12C 1/02, C12C 1/067, C12N 1/14

(54) **ENSEMENCEMENT PAR GEOTRICHUM CANDIDUM AU COURS DU MALTAGE DE CEREALES OU AUTRES VEGETAUX**
BEIMPFEN MIT GEOTRICHUM CANDIDUM BEIM MÄLZEN VON ZEREALEM KORNGUT ODER ANDEREN PFLANZEN
INOCULATION BY GEOTRICHUM CANDIDUM DURING MALTING OF CEREALS OR OTHER PLANTS

(30) Priorité: 24.04.1995 FR 9505038
(43) Date de publication de la demande: 04.03.1998
(73) Titulaire: Institut Francais Des Boissons De La Brasserie Malterie, 54712 Vandoeuvre (FR)
(72) Inventeur: BOIVIN, Patrick, F-54580 Messein (FR); MALANDA, M'Baka, F-54000 Nancy (FR)
(74) Mandataire: Vaillant, Jeanne
(86) Numéro de dépôt international: FR9600621
(87) Numéro de publication internationale: WO9634085

(56) Documents cités:
- WO-A-94/16053
- APPLIED BIOCHEMISTRY AND MICROBIOLOGY, vol. 15, no. 6, PLENUM PUBLISHING CORPORATION, pages 670-673, XP002011810 MARICHENKO, V.A. ET AL.: "Effect of fungal cellulases on enzymic activity of malt."
- JOURNAL OF THE INSTITUTE OF BREWING, vol. 94, LONDON GB, pages 85-88, XP002011811 DOUGLAS, P.E. ET AL.: "A microbiological evaluation of barley malt production."

## Description

L'invention a trait à l'application d'un micro-organisme donné dans le procédé de maltage de céréales ou autres végétaux, tel que blé, orge, riz, sorgho, maïs, sarrasin pour le contrôle du développement de la microflore à même de contaminer et coloniser ces céréales ou autres végétaux.

La présente invention trouvera son application dans le domaine de la malterie et, par conséquent, auprès de brasseurs-malteurs mais aussi dans le cadre des distilleries et, en général, dans toute industrie utilisant des céréales ou des végétaux maltés.

Il convient d'observer que les céréales ou autres végétaux, par exemple, l'orge avant même de subir les différentes opérations de traitement et de transformation au niveau d'une unité de maltage sont contaminés, naturellement, par de nombreux micro-organismes. Ainsi, dans l'exemple particulier de l'orge, celui-ci est l'hôte naturel d'une flore importante et diverse qui se compose, principalement, de bactéries, de levures ainsi que de champignons filamenteux. En fait, cette flore contamine et colonise le grain d'orge tant dans le champ que lors du stockage. La nature et le nombre de ces micro-organismes dépendent, bien sûr, des conditions de culture et du climat, tout comme de la durée et des conditions de stockage.

Les différentes analyses effectuées au niveau de ces micro-organismes issus de champs a permis de répertorier une centaine d'espèces environ. Toutefois, les moisissures les plus rencontrées sont Altemaria, Clasdosporium et Fusarium.

L'on a, par ailleurs, observé qu'en cas de récolte tardive ou lorsque celle-ci s'effectue par temps humide, les grains présentent une contamination importante par Fusarium. Quant à la flore qui contamine et colonise les grains pendant leur stockage, donc avant maltage, elle est composée par des champignons filamenteux xérophiles. Là-encore, l'étude de ces champignons filamenteux a permis de mettre en évidence que les espèces prédominantes sont Aspergillus et Pénicilium.

En fin de compte, le problème réside dans le fait que les différentes opérations qui consistent à transformer les céréales, notamment, en malt, s'effectuent dans des conditions qui favorisent le développement de la microflore présente préalablement sur le grain. Effectivement, l'on retrouve, lors du maltage, des conditions notamment sur le plan de l'humidité, de la température, des nutriments ou encore du point de vue du temps de séjour dans les différents réacteurs qui, finalement permettent aux bactéries et aux levures de se multiplier dans une échelle allànt de 10² à 10⁴ sans compter un développement important des moisissures.

Il convient, à ce propos, de rappeler les différentes étapes du procédé de maltage. Plus précisément, après préparation du grain, celui-ci subit une opération de trempe d'une durée d'une quarantaine d'heures environ, ceci afin de réaliser un apport de 35 à 45 % d'humidité. Suit, alors, la germination pendant une durée d'environ six jours à une température de l'ordre de 16°. Après cela, l'on procède au touraillage qui consiste à sécher le grain à différents paliers de température, celle-ci évoluant entre 50° et 80° Celsius environ. Il s'ensuit le dégermage et le stockage du malt finalement obtenu.

Ce développement, non contrôlé de la microflore constitue un inconvénient dans la mesure où il influe la qualité du malt et, bien sûr, du produit final, par exemple, de la bière, qui en est issue. En effet, il est à l'origine d'une production d'inhibiteurs de la germination. Par ailleurs, il en résulte une production d'enzymes indésirables tels que des lipases, des oxydases, protéases, etc... Certains de ces micro-organismes présents en quantité surabondante sont également à l'origine de flaveurs indésirables. La bière finalement obtenue peut, ainsi, présenter un goût de moisi ou encore de terre ou d'oxydé.

De manière générale, dans le domaine de la brasserie, l'on redoute le phénomène du giclage qui, tel que l'indique son nom, consiste en ce que la bière gicle hors de la bouteille au moment de l'ouverture, ceci en raison d'une instabilité du gaz carbonique. En fait, ce sont des micro-organismes spécifiques qui en sont à l'origine. Il convient, par conséquent, d'éviter leur présence ou tout au moins que leur quantité ne dépasse pas un seuil donné; Or, la seule manière d'aboutir, de manière certaine, à ce résultat, consiste à éviter le développement important de la microflore et, donc, des précurseurs au giclage, produits notamment par Fusarium.

A cela l'on peut ajouter que la présence de certains micro-organismes tels que les bactéries du type Pédiococcus a des conséquences sur la vitesse de filtration de la maische et conduit à un colmatage rapide des filtres.

Toutefois, l'inconvénient majeur rencontré au travers du développement important des moisissures, consiste en une augmentaton considérable du risque de formation de mycotoxines sachant que des moisissures toxicogènes sont naturellement présentes sur les céréales et les végétaux.

En fait, malgré les inconvénients précités, ce développement de la microflore n'est guère contrôlé dans les malteries. La faute en est à l'absence de moyens efficaces permettant, effectivement, ce contrôle.

Ainsi, seule une opération de désinfection peut être effectuée au moment de la trempe, ceci par l'adjonction d'eau de javel ou de lait de chaux. Si l'ajout de tels désinfectants n'a que peu d'influence sur la microflore, il n'en présente pas moins un risque pour certaines opérations du procédé de maltage. Notamment en cas de dépassement de doses bien déterminées, ces désinfectants représentent un risque pour la germination des grains et peuvent conduire à des problèmes de faux goût dans la bière par exemple.

La présente invention a pour but de remédier à l'ensemble de ces inconvénients en évitant le développement, plus particulièrement, de la microflore indésirable, ceci au travers de l'ensemencement d'un micro-organisme spécifique ayant été sélectionné en raison des résultats probants qu'il permet d'obtenir.

Il a été proposé d'utiliser dans un procédé de maltage, un ensemencement de ferment comprenant Geotrichum Candidum pour contrôler le développement de la microflore.

L'utilisation de bactéries lactiques, qui est répandue dans les procédés agro-alimentaires et même en malterie pour produire des malts acides, a été proposée pour réduire le développement de la flore fongique, en particulier de Fusarium (WO 94/16053). Cependant, l'ensemencement de bactéries lactiques au cours du maltage ne permet qu'une inhibition partielle du développement de la flore fongique et ne permet pas d'inhiber la synthèse de mycotoxines au cours du procédé de maltage.

Geotrichum candidum qui est présent naturellement sur l'orge en cours de germination et sur le malt fini a été proposé, d'une part, pour inhiber le développement de la flore toxicogène au cours du maltage et la synthèse de mycotoxines, d'autre part, pour améliorer la qualité biochimique et physico-chimique du malt.

Geotrichum candidum est très fréquent dans l'environnement laitier. La plupart des souches ont été isolées du lait et du fromage. Il fait partie de la flore naturellement présente dans la fabrication des fromages au lait cru. Longtemps dans l'industrie laitière, on a cherché à éviter le développement de ce champignon levureforme qui, mal maîtrisé, est susceptible de provoquer des défauts d'aspect du fromage. Depuis quelques années, nous assistons, de la part des industriels fromagers à un regain d'intérêt pour Geotrichum candidum en raison de son rôle dans l'amélioration des qualités organoleptiques des fromages et l'inhibition du développement de bactéries pathogènes et de moisissures indésirables de l'affinage.

L'activité lipasique de ce micro-organisme, recherchée en fromagerie, était considérée comme un inconvénient majeur dans le domaine de la malterie, notamment pour les altérations organo-leptiques qu'il pouvait entraîner (Drost et al. ASBC Journal, 1990, 124-131; Kobayashi et al. Journal of fermentation and bioengineering, 1993, 371-375).

Ainsi, l'invention concerne l'utilisation d'un micro-organisme de la famille de Geotrichum candidum dans le procédé de maltage de céréales ou autres végétaux tels que blé, orge, riz, sorgho, maïs, sarrasin pour le contrôle du développement de la flore à même de contaminer et coloniser ces céréales ou autres végétaux.

De plus, selon l'invention, des souches sélectionnées de Geotrichum candidum sont ensemencées en cours de maltage, soit sous forme de culture pure, soit sous forme de culture mixte en association avec un ou plusieurs autres micro-organismes, notamment des bactéries lactiques.

Selon l'invention, l'ensemencement de Geotrichum candidum s'effectue dans des proportions comprises entre 10⁵ et 10⁹ de préférence entre 10⁷ et 10⁸ unités formant des colonies (UFC) par kilogrammes de céréales ou autres végétaux, ceci de manière à obtenir le résultat recherché à savoir le contrôle du développement de la microflore.

Les avantages obtenus grâce à cette invention consistent, essentiellement, en ce que le Geotrichum candidum a un effet inhibiteur de la flore indésirable telle que du Fusarium, Pénicilium, Aspergillus, sans compter qu'il permet d'inhiber la production de métabolites secondaires de la flore indésirable, telles que les mycotoxines et les précurseurs du giclage.

L'invention concerne aussi l'utilisation de ce micro-organisme dans le maltage de céréales ou autres végétaux, tels que blé, orge, riz, sorgho, maïs, sarrasin pour le contrôle du phénomène de giclage de la boisson obtenue à partir du malt.

Il convient d'observer, en outre, que cette amélioration de l'état sanitaire du malt s'accompagne d'apports qualitatifs, puisque l'on obtient, au travers de cet ensemencement de Geotrichum candidum une réduction des acides gras du moût qui, par exemple, sont souvent responsables de problèmes de faux goût dans la bière, tels que le goût d'oxydé, et d'une mauvaise tenue de la mousse. De plus, l'inhibition par Geotrichum candidum de certaines flores responsables de la synthèse de polysaccharides entraîne une amélioration du point de vue de la cinétique de filtration.

Finalement par l'emploi de certaines souches de Geotrichum candidum, il est possible de favoriser le développement de bactéries lactiques qui sont en mesure de renforcer l'effet inhibiteur de la flore indésirable et d'améliorer la qualité du malt par un abaissement naturel du pH.

Les inventeurs ont pu observer la disparité des effets des différentes souches de Geotrichum candidum sur l'inhibition de la microflore, sur l'inhibition de la production de métabolites secondaires de la flore indésirable : mycotoxines et précurseurs du giclage, ainsi que sur les propriétés organolyptiques ou les qualités physico-chimiques de la bière.

Par qualité physico-chimique, on entend notamment:
- la réduction des acides gras du moût qui sont souvent à l'origine des problèmes de faux goût dans la bière, tel que le goût de l'oxydé ou encore d'une mauvaise tenue de la mousse ;
- une meilleure filtration de la maische grâce à l'inhibition de certaines flores responsables de la synthèse de polysaccharides ;
- l'absence du phénomène de giclage.

La présente invention porte sur les moyens de sélection des souches de Geotrichum candidum présentant à la fois les qualités inhibitrices décrites ci-dessus, et l'absence des inconvénients, essentiellement de type organoleptiques résultant de la faible activité lipasique.

Les moyens, ou procédés de sélection desdites souches, comprennent une sélection des souches naturelles prélevées sur le malt selon une combinaison de critères qualitatifs et productifs, certains de ces critères étant obligatoires, d'autres étant, combinés aux critères obligatoires, fortement recommandés, cette sélection intervenant après l'isolement et le clonage des souches naturelles.

Les critères obligatoires sont :
a) : une faible activité lipasique,
b) : l'inhibition de la flore indésirable se développant au maltage,
c) : l'obtention d'une souche ni mutagène ni toxicogène.

Les souches préférées sont celles qui, en outre, présentent:
d) : une stabilité de la solution de spores supérieure à 6 mois,
e) : une production abondante de spores de 10⁷ à 10⁸/ml,
f) : une amélioration de la vitesse de filtration maische,
   ou la combinaison de ceux-ci.

La synthèse de lipase au cours du maltage doit être la plus faible possible car cette enzyme peut conduire à des problèmes de goûts d'oxydé dans la bière. Le potentiel lipasique des souches de Geotrichum candidum sélectionnées pour leur utilisation en malterie doit être le plus faible possible pour qu'il n'y ait pas synthèse de lipase exogène au cours du procédé de maltage.

La présente invention porte également sur les souches de Geotrichum Candidum susceptibles d'être obtenues par un procédé de sélection tel que décrit ci-dessus et comprenant au moins les critères a), b), c) et de préférence, combinés aux précédents, les critères e), f) ou g), ou la combinaison de ceux-ci.

Les souches susceptibles d'être ainsi obtenues ont de préférence une activité lipasique inférieure à 2.5.10⁻⁷ nKat/cellules, entraînent une inhibition totale de la flore indésirable et ne présentant pas de pouvoir mutagène.

En outre, la production de spores doit atteindre préférentiellement 10⁷ spores/ml, et de façon encore préférée se situer entre 10⁷ et 10⁸ par ml.

Le procédé de sélection a permis de sélectionner des souches de Geotrichum candidum qui procurent, en la matière, de très bons résultats que ce soit d'un point de vue de l'inhibition de la flore indésirable ou de l'amélioration de la qualité du malt qui s'avère, par ailleurs, significative, et se traduit par une meilleure filtration de la maische et un rendement supérieur en extrait. Des souches telles qu'obtenues par ce procédé ont été déposées le 6 septembre 1994 à la Collection Nationale de Culture de Micro-organismes auprès de L'Institut Pasteur et ont été enregistrées sous le n° I-1475 et n° I-1474. Au cours d'essais ayant consisté à ensemencer l'une de ces souches de Geotrichum candidum dans des proportions comprises entre 10⁷ et 10⁸ (UFC) par kilogramme d'orge, ceci dès le premier sous eau de trempe, dans le cadre du maltage d'orge « six rangs d'hiver » correspondant, par conséquent, à une orge de qualité moyenne, a permis de démontrer qu'il était possible d'obtenir, à l'issue, un malt d'une très bonne qualité.

Dans l'ensemble du texte, et en particulier dans les exemples, la souche I-1474 est la souche GC3 ; la souche I-1475 est la souche GC4.

Il est surprenant de constater que ces avantages découlant de l'invention sont obtenus sans conséquence, ni sur le procédé de fabrication de la bière, ni sur la bière qui en résulte. Plus précisément, aucune variation significative du procédé n'a été constatée. De plus, la bière obtenue à partir de malt traité au Geotrichum candidum est analogue d'un point de vue organoleptique à la bière issue de malt non traité. Le profil aromatique est identique et en dégustation triangulaire il n'y a guère de différence.

D'autres buts et avantages de la présente invention apparaîtront au cours de la description qui va suivre exposant les méthodes d'ensemencement de Geotrichum candidum.

La compréhension de cette description sera facilitée en se référant au dessin joint en annexe et dans lequel :
- la figure 1 expose les grandes étapes de la malterie ; de plus ont été représentées par des flèches les étapes au cours desquelles l'on peut procéder à un ensemencement de micro-organismes sélectionnés ;
- la figure 2 montre l'évolution de l'activité lipasique au cours du maltage selon qu'il y a ou non ensemencement contrôlé de Geotrichum candidum ;
- la figure 3 est un diagramme illustrant la cinématique de filtration selon qu'il y a ou non ensemencement de Geotrichum candidum.

La présente invention a pour objectif d'intervenir au niveau du procédé de maltage en vue d'améliorer l'état sanitaire et qualitatif du malt finalement obtenu à partir de céréales ou autres végétaux, tels que le blé, l'orge, le riz, le sorgho, le maïs, le sarrasin.

La présente description s'attachera, plus particulièrement, à la transformation de l'orge en malt, mais il est évident que la présente invention n'est nullement limitée par une telle application et qu'elle s'étend à toutes céréales ou aux végétaux susceptibles de subir un procédé de maltage.

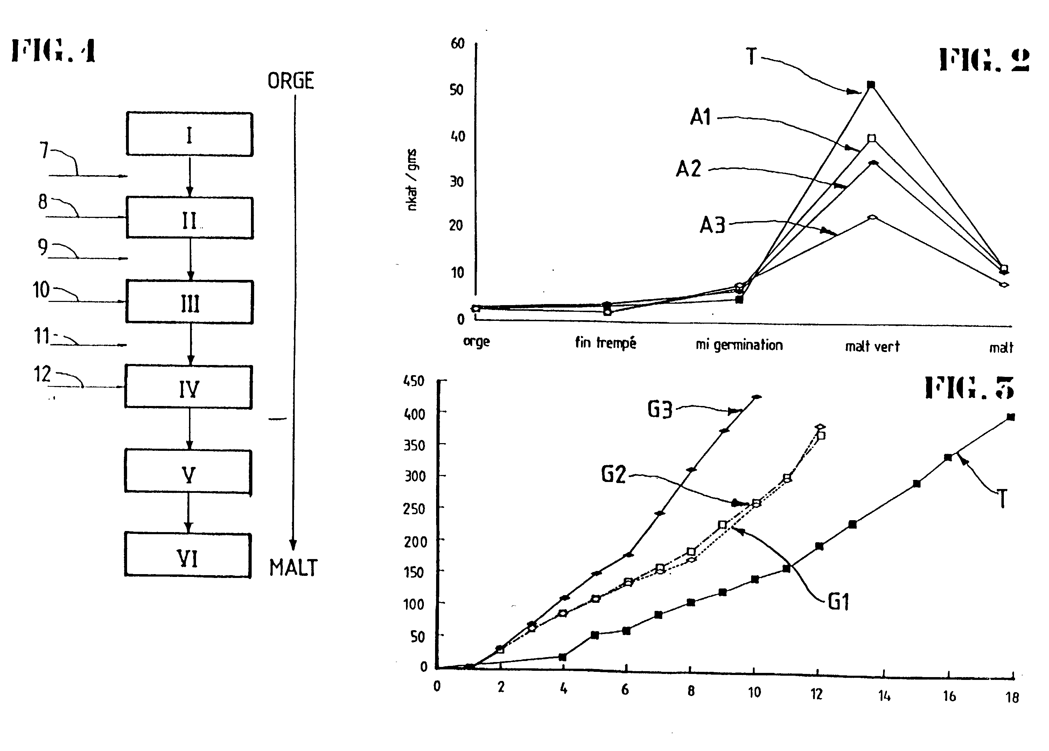

La figure 1 illustre, de manière schématisée, les six étapes I, II, III, IV, V, VI qui définissent ce procédé de maltage. Ainsi, après préparation du grain correspondant à la première étape I, l'orge subit une opération de trempe qui consiste à plonger l'orge dans l'eau afin d'augmenter sa teneur en humidité pour que celle-ci atteigne 35 à 45 %. A noter qu'au cours de cette opération de trempe, l'eau est renouvelée une ou plusieurs fois, pour assurer une action de lavage et d'oxygénation du grain.

Après cette seconde étape Il, l'orge ainsi humidifié, passe à l'étape III qui est celle de la germination. Il s'ensuit le touraillage (étape IV) qui consiste à sécher le grain en le soumettant à des paliers de température entre 50° Celsius et 80° Celsius et en respectant, à chacun des paliers, des durées de séchage déterminées.

Puis vient l'étape V qui consiste au dégermage. Le malt qui en résulte est alors stocké ce qui constitue la dernière étape VI de ce procédé de maltage.

L'on comprend, aisément, que l'apport d'humidité, les temps de séjour et les températures auxquelles sont soumis les céréales ou autres végétaux au cours de ces différentes étapes de mattage constituent des conditions favorables au développement de la microflore déjà présente avant maltage.

La présente invention a pour but d'empêcher le développement de la microflore notamment indésirable par l'ensemencement d'un micro-organisme particulièrement bien adapté à cet effet. En fait, selon le micro-organisme qui, dans le cadre de l'application concernée, présente, les résultats souhaités, est issu de la famille de Geotrichum candidum.

A ce propos, dans la figure 1 sont repérés par des flèches 7 à 12, les moments au cours du procédés de maltage où cet ensemencement peut avoir lieu.

Ainsi, Geotrichum candidum peut être ensemencé et homogénéisé par aération dans la première eau de trempe avant l'introduction de l'orge dans la cuve. L'ensemencement peut être effectué directement sur l'orge arrivant dans la cuve de trempe par pulvérisation. Toutefois, il peut être préféré, d'effectuer cet ensemencement, lors de cette première étape de sous-eau, mais au cours des deux ou trois sous-eaux, surtout si l'orge est fortement contaminée.

Cet ensemencement peut également avoir lieu lors du transport de l'orge entre l'unité de trempe à celle de germination et à ce stade du procédé de pulvériser sur le grain d'orge de l'eau ou des activateurs de germination. A noter que cela ne pose aucun problème, puisqu'il est, habituellement, prévu dans les unités de maltage et à ce stade du procédé de pulvériser sur le grain d'orge des activeurs de germination.

Finalement, et tel que représenté dans la figure 1, cet ensemencement peut avoir lieu, également, en cours de germination, lors du transfert en direction du touraillage ou au moment même de sécher le grain.

### EXEMPLE I: Procédé de sélection des souches de Geotrichum candidum.

Le tableau I représente l'activité lipasique en nanomoles par seconde par cellule de différentes souches de Geotrichum candidum après une incubation d'une semaine à 20°C dans un milieu d'extrait de malt. Les souches L+ et L- sont des souches de Geotrichum candidum isolées de laiteries et fromageries représentant les extrêmes en potentiel lipasique. Les autres souches ont été isolées de malterie. On constate que les souches « malteries » ont un potentiel lipasique faible, inférieur aux souches « laiteries ». Ce test en milieu liquide permet de sélectionner les souches de Geotrichum candidum à faible potentiel lipasique.

**TABLEAU I :**

| Souches | L⁺ | L⁻ | GC1 | GC2 | GC3 |
|---|---|---|---|---|---|
| Activité lipasique (10⁻⁷ nKat/cellule) | 6.4 | 2.5 | 0.3 | 1.7 | 1.5 |

Le second test a pour but de montrer l'effet de différentes souches sélectionnées sur l'activité lipasique totale dans le grain exprimé en nanomoles par seconde et par gramme de matière sèche au cours du maltage selon qu'il y a ou non ensemencement de Geotrichum candidum.

Dans le tableau II suivant, sont exprimés les résultats de ce test.

**TABLEAU II :**

| Souches | TEMOIN | GC1 | GC2 | GC3 | GC4 | GC5 |
|---|---|---|---|---|---|---|
| Activité lipasique sur malt vert nKAT/gms | 19.9 | 15.1 | 16.9 | 14.8 | 11.0 | 11.0 |

On constate par ce test un écart de l'activité lipasique totale sur malt vert, résultant de l'activité de l'orge et du développement de la microflore. L'écart entre l'essai sans ensemencement et avec ensemencement de différentes souches résultent d'une part de la synthèse de lipase par Geotrichum candidum, du nombre de cellules présentes sur le malt vert et l'inhibition par Geotrichum candidum d'une flore à potentiel lipasique. On contaste que les souches testées dans cet exemple conduisent à une activité lipasique sur le malt vert plus faible que le témoin. Ce test permet de sélectionner les souches de Geotrichum candidum conduisant à une faible activité lipasique sur malt vert.

Le développement non contrôlé de certaines flores au cours du maltage peut entraîner une variation de la performance de la filtration maïsche, attente qualitative importante pour le brasseur. La diminution de la performance de la filtration peut être due à la synthèse de polysaccharides, l'amélioration due soit à la synthèse d'enzymes cellulolytiques (β-glucanases et pentosanases) soit à l'inhibition de la flore responsable de la synthèse de polysaccharides. Comme Geotrichum candidum peut avoir différentes actions, nous avons utilisé, pour la sélection de souches répondant à cette attente du brasseur, un test permettant de quantifier la performance de la filtration du malt, dénommé « Filtration TEPRAL ». Le tableau III donne les valeurs de cinétique de filtration et lavage de la maische de différents malts obtenus avec et sans ensemencement de Geotrichum candidum au cours du maltage. GC1, GC2, GC3 et GC5 sont les différentes souches de Geotrichum candidum testées.

**TABLEAU III :**

| Souche | Témoin sans ensemencement | GC1 | GC2 | GC3 | GC5 |
|---|---|---|---|---|---|
| cinétique de filtration (g/mn) | 23.2 | 27.6 | 28.8 | 40 | 8.8 |
| cinétique de lavage (g/mn) | 42 | 42.8 | 40 | 56.4 | 10.4 |

Comme le montre le tableau III, la performance de la filtration maische dépend de la souche de Geotrichum candidum utilisée. Les souches qui retardent la filtration de la maische seront éliminées.

La productivité de Geotrichum candidum ainsi que leur stabilité au cours du temps est un paramètre économique important pour la sélection des souches. Des tests de productivité en fermenteur et de stabilité à 4°c ont été développés. Le tableau IV indique les valeurs de productivité et de stabilité de différentes souches de Geotrichum candidum.

**TABLEAU IV :**

| Souche | GC1 | GC2 | GC3 | GC4 | GC5 |
|---|---|---|---|---|---|
| productivité nombre de spores par ml (x10⁶) | 20 | 100 | 50 | 90 | 22 |
| % de spores viables après 8 semaines de stockage à 4°C | 80 % | 8 % | 50 % | 90 % | 100 % |

Les souches de Geotrichum candidum ont une productivité et stabilité variables.

L'inhibition de la flore indésirable se développant au maltage, est vérifiée pour les différentes souches testées.

Ces tests sont complétés par un test de toxicologie et de mutagénicité des souches.

Ces différents tests ont permis de sélectionner des souches de Geotrichum candidum qui procurent, en la matière, de très bons résultats que ce soit d'un point de vue de l'inhibition de la flore indésirable ou de l'amélioration de la qualité du malt qui s'avère d'ailleurs significative et se traduit par une activité lipasique plus faible et une meilleure filtration de la maische.

### EXEMPLE II : Effet de l'ensemencement par Geotrichum candidum sur le développement des microflores :

L'avantage d'effectuer un ensemencement précoce consiste en ce que l'on contrôle, dès l'origine, le développement de la microflore.

Des expériences ont démontré qu'un ensemencement dans des quantités de l'ordre de 10⁵ à 10⁹ préférentiellement entre 10⁷ à 10⁸ unités formant des colonies (UFC) par kilogramme de céréales ou autres végétaux permet d'obtenir des résultats probants. A ce propos, cet ensemencement peut s'effectuer en une seule ou en plusieurs fois au cours des étapes précédemment mentionnées. De plus, Geotrichum candidum peut être ensemencé sous forme de culture pure ou sous forme de culture mixte, c'est-à-dire en association avec d'autres micro-organismes, tels que des bactéries lactiques. Finalement, un ensemencement en alternance de culture pure de Geotrichum candidum ou de culture mixte, puis de culture autre que Geotrichum candidum constitue, également, une solution envisageable.

Il convient d'observer, à ce propos, qu'un ensemencement mixte ou alterné permet, tout en garantissant la qualité sanitaire du malt par un contrôle strict du développement des micro-organismes, d'améliorer d'autres propriétés. Ainsi, cela peut se traduire sous forme d'une amélioration du rendement en extrait ou encore d'inhibition d'enzymes indésirables, telles que les oxydases ou au contraire la production d'enzymes intéressantes, telles que les cellulases. L'on peut également rechercher à modifier la flaveur du malt en associant à Geotrichum candidum ou en procédant à un ensemencement alterné avec Geotrichum candidum, d'un micro-organisme spécifique permettant d'obtenir le résultat recherché.

Le tableau V ci-dessous illustre les avantages découlant de la présente invention :

**TABLEAU V :**

| Moisissures | Orge Fusariée | Malt « témoin » | Malt « Geotrichum » |
|---|---|---|---|
| | % DE GRAINS CONTAMINES | | |
| ALTERNARIA | 20 | 5 | 0 |
| ASPERGILLUS | 20 | 15 | 0 |
| FUSARIUM | 100 | 40 | 5 |
| PENICILIUM | 30 | 10 | 5 |
| GEOTRICHUM | 0 | 0 | 100 |

Ainsi, dans ce tableau V sont reportés les pourcentages de grains contaminés par les moisissures les plus communément rencontrées dans ce domaine notamment Alternaria, Aspergillus, Fusarium, Penicillium et Geotrichum, ceci selon que les mesures sont effectuées sur de l'orge fusariée, sur du malt témoin c'est-à-dire n'ayant subi aucun ensemencement et, finalement sur du malt Geotrichum c'est-à-dire du malt issu d'un procédé de maltage au cours duquel il y a eu ensemencement de Geotrichum candidum. Les résultats sont, bien sûr, flagrants et révélateurs dans la mesure où, dans le malt « Geotrichum » seuls 5 % de grains sont contaminés par Fusarium et Penicillium, ces chiffres étant à comparer au malt témoin dans lequel l'on retrouve 40 % de grains contaminés par Fusarium et environ 10 % par Penicillium..

### EXEMPLE III : Effet de Geotrichum Candidum sur la production de mycotoxines lors du maltage :

Le tableau VI, ci-après, a pour but de montrer l'effet inhibiteur de Geotrichum candidum sur la production de mycotoxines lors du maltage. Plus précisément, cette démonstration est effectuée au travers du dosage de la Zeralenone au niveau de l'orge fusariée, du malt témoin, c'est-à-dire n'ayant subi aucun traitement et, finalement, du malt Geotrichum.

**TABLEAU VI :**

| | Orge fusariée | Malt témoin | malt « Geotrichum » |
|---|---|---|---|
| Zeralenone µg/kg | 3 | 100 | 3 |

### EXEMPLE IV : Effet de l'ensemencement de Geotrichum sur l'activité lipasique :

Les avantages découlant de l'invention apparaissent, également, au travers du diagramme illustré dans la figure 2 représentant l'évolution de l'activité lipasique au cours du maltage selon qu'il y a ou non ensemencement de Geotrichum candidum. Au niveau de ce diagramme sont représentées, en abscisse, les étapes du procédé de maltage, tandis qu'en ordonné on retrouve l'activité lipasique totale dans le grain en nanomoles par seconde par gramme de matière sèche. Ainsi, l'on constate un écart notamment au stade du malt vert, entre la courbe T correspondant au malt témoin c'est-à-dire non traité et les courbes A1, A2 et A3 correspondant à des situations où il y a ensemencement de différentes souches de Geotrichum candidum dénommées A1, A2 et A3.

Ainsi, il est clair que l'ensemencement de souches sélectionnées de Geotrichum candidum permet réellement de contrôler l'évolution de la microflore et que l'on obtient bien des améliorations sanitaires souhaitées, à savoir :
- inhibition de la flore indésirable : Fusarium, Pénicillium. Aspergillus...;
- inhibition de la production de métabolites secondaires de la flore indésirable : mycotoxine et précurseur du giclage ;
- développement accru des bactéries lactiques qui peuvent renforcer l'effet inhibiteur de la flore indésirable et améliorer la qualité du malt par un abaissement naturel du pH.

En fait, les résultats issus de l'invention sont d'autant plus intéressants que cette amélioration sanitaire est accompagnée d'apports qualitatifs majeurs tels que :
- la réduction des actifs gras du moût qui sont souvent à l'origine des problèmes de faux goût dans la bière, tel que le goût de l'oxydé ou encore d'une mauvaise tenue de la mousse ;
- meilleure filtration de la maische grâce à l'inhibition de certaines flores responsables de la synthèse de polysaccharides.

### Exemple V : Effet de l'ensemencement de Geotrichum candidum sur la filtration de la maische :

L'amélioration obtenue sur le plan de la filtration est illustrée sur le diagramme de la figure 3 représentant la cinétique de filtration déterminée selon une méthode dénommée « BRASSIN TEPRAL ». Ainsi, dans ce diagramme, l'on retrouve, en abscisse, le temps en minutes, tandis qu'en ordonné, est reportée la quantité de moût filtré en grammes. La courbe T, correspondant au malt témoin, n'ayant subi aucun traitement, se situe largement en-dessous des courbes GC1, GC2 et GC3 correspondant à des malts ayant été ensemencés par différentes souches de Geotrichum candidum GC1, GC2 et GC3.

### EXEMPLE VI : Effet de l'ensemencement par Geotrichum Candidum sur la qualité des effluents :

L'on a pu observer que l'ensemencement de Geotrichum candidum, tout particulièrement pendant cette étape de la trempe correspondant au procédé de maltage, avait également des répercussions bénéfiques sur les effluents. En effet, l'analyse des eaux éliminées après la trempe montre que l'ensemencement de Geotrichum candidum permet une diminution de la demande chimique en oxygène « DCO ».

Le tableau VII ci-après illustre, tout particulièrement, les résultats obtenus suite à l'ensemencement de Geotrichum candidum correspondant à la souche I-1475 dans des proportions de 10⁷ environ, (UFC) par kilogramme d'orges, l'orge maltée étant du type « deux rangs de printemps ».

Le procédé de maltage mis en oeuvre était de type standard, c'est-à-dire que le diagramme de trempe qui a été respecté est le suivant :
- 1er sous eau 5 heures suivi de 7 heures sous air,
- 2ème sous eau 5 heures suivi de 7 heures sous air,
- 3ème sous eau 5 heures suivi de 7 heures sous air.

**TABLEAU VII :**

| Analyses des eaux de trempe | | |
|---|---|---|
| | DCO (mg 0₂/litre) | |
| | Témoin | I-.1475 |
| 1ère sous eau | 969 | 633 |
| 2ème sous eau | 691 | 614 |
| 3ème sous eau | 883 | 336 |

Il est évident, au travers de ce tableau, que les résultats obtenus sont significatifs comparés aux témoins correspondant à de l'orge maltée sans ensemencement de Geotrichum candidum.

En conséquence, l'application de Geotrichum candidum, à la malterie, conformément à l'invention, conduit à un net progrès dans le domaine considéré.

Bien que l'invention ait été décrite à propos d'une forme de réalisation particulière, il est bien entendu qu'elle n'y est nullement limitée et qu'on peut y apporter diverses modifications de formes, de matériaux et de combinaisons de ces divers éléments, sans pour cela s'éloigner du cadre et de l'esprit de l'invention.

### EXEMPLE VII : Toxicité de Geotrichum Candidum par test d'Ames et sur embryon de poulet :

Deux isolats fongiques répertoriés GC3 et GC4 ont été étudiés.

### Protocole expérimental :

Préparation des extraits de cultures : Les deux isolats GC93133 et GC93201 ont été cultivés sur milieu semi-synthétique (type Czapek) glucosé, liquide (technique de P. Lafont et J. Lafont, Ann. Inst. Pasteur, 1970, 118, 340-348) et, parallèlement, sur le même milieu liquide, gélosé. Après incubation (6 jours à -25°C), les cultures en milieu liquide, in toto (mycélium et liquide sous-jacent), ont été extraites par le chloroforme à reflux (3 fois successivement 1 vol. de chloroforme). Après séparation, les trois phases chloroformiques ont été mélangées, filtrées sur Whatman, déshydratées et évaporées à siccité sous azote. Le résidu d'évaporation a été délipidé par lavage à l'éther de pétrole (extrait A). La phase aqueuse, après filtration, a été concentrée sous vide à température inférieure à 45°C (extrait B).

Le mycélium développé en milieu gélosé a été broyé en solution aqueuse de KC1 à 5 % (p:v) acidifiée (SO₄H₂, 20 ml/L) ; 1 vol. de ce broyat a été additionné de 7 vol. d'acétonitrile ; après un séjour de 18 heures à 4°C, la suspension a été filtrée, délipidée par lavages par l'éther de pétrole et soumise à deux extractions successives à température du laboratoire par le chloroforme. La phase chloroformique a été évaporée à siccité sous azote (extrait C).

### Essais de toxicité sur embryon de poulet :

Les essais ont utilisé un lot homogène d'oeufs (ponte d'une même journée dans un même couvoir). L'inoculation des produits à tester a été faite selon la méthodologie décrite par P. Lafont et J. Lafont (Bull, Acad. Vét. Fr., 1979, 52, 119-124). l'incubation des oeufs a été poursuivie jusqu'à l'éclosion, le développement de l'embryon étant observé par mirage hebdomadaire.

Dans le cas des extraits A et C, l'inoculum a été préparé aseptiquement en alcool éthylique / eau (50/50, v/v) ; dans le cas de l'extrait B, le concentré de la phase aqueuse a été stérilisé par filtration sur membrane Millipore G.

### Test de génotoxicité sur bactéries (test de Ames) :

Les souches hétérotrophiques de Salmonella typhimurium répertoriées TA1535, TA1538, TA98 et TA10 ont été utilisées. La méthodologie appliquée a été celle décrite par B.N. Ames, J. McCann et E. Yamasaki (Mut. Res., 1975, 31, 347-364) tant en matière de manipulations bactériologiques que pour l'obtention de préparations de microsomes de foie de rat (animaux traités au phénobarbital).

Les extraits A et C ont été incorporés dans la couche superficielle des milieux gélosés sous forme de solutions en diméthylsulfoxide préparées aseptiquement ; les extraits B ont été stérilisés par filtration avant leur incorporation.

Des témoins « positifs » ont été obtenus en employant comme agents génotoxiques l'aflatoxine B₁ et le 3,4-benzopyrène.

### Résultats :

### Toxicité sur embryon de poulet :

Pour chaque oeuf, l'inoculum correspondait à 15 mg (poids sec) de mycélium dans le cas des extraits A et C, à 1 ml de culture dans le cas de l'extrait.

Pour les différents lots d'oeufs, le taux d'éclosion est rapporté dans le tableau VIII. Aucune différence significative n'a été mise en évidence entre les lots témoins et ceux ayant reçu les extraits des cultures, ni entre les deux isolats.

**TABLEAU VIII :**

| Taux d'éclosion (%) | | | |
|---|---|---|---|
| | Extrait A | Extrait B | Extrait c |
| GC 93133(*) | 95 | 100 | 90 |
| GC93201 (**) | 90 | 90 | 95 |
| Témoins non inoculés (**) | | | 92,5 |
| Témoins inoculés \| solvants | des extraits | A et C (*) | 95 |
| \| (*) eau | distillée | | 90 |

| | | | |
|---|---|---|---|
| (*) lot de 20 oeufs | | | |
| (**) lot de 40 oeufs. | | | |

Aucune malformation n'a été observée sur la totalité des poussins.

### Test de Ames :

Deux essais successifs ont été effectués en utilisant les concentrations suivantes d'extraits (rapportées au poids sec de mycélium ou au volume de culture) par ml de milieu gélosé dans lequel étaient cultivées les souches bactériennes-tests :
- extraits A et C : 20 et 200 mg
- extrait B : 1 et 10 ml.

Dans ces conditions expérimentales les extraits des deux isolats de Geotrichum candidum n'ont montré aucun effet sur la fréquence de réversion chez les souches de Salmonella typhimurium, alors que les nombres de colonies de revenants, par boîte de Petri, étaient compris, suivant les souches, entre 49 (+ 12) et 195 (+ 24) dans le cas de l'aflatoxine B₁ à la concentration de 1µ//ml, entre 28 (+ 10) et 218 (+ 41) dans celui du 3,4-benzopyrène à la concentration de 0,2 µg//ml.

De plus, des opérations de contrôle ont indiqué que les extraits des cultures des deux isolats de Geotrichum candidum n'avaient pas d'effet antibiotique vis-à-vis des souches de Salmonella.

Nous pouvons conclure que les essais rapportés ci-dessus indiquent l'absence de production in vitro pour les deux isolats GC3 et GC4, de substances toxiques et tératogènes pour un vertébré et de substances présentant des activités génotoxiques.

## Revendications

1. Souches de microorganismes de la famille de Geotrichum candidum susceptibles d'être obtenues par sélection puis clonage de souches naturelles du micro-organisme et présentant à la fois :
- une activité lipasique inférieure à 2.5.10⁻⁷ (nanomoles par seconde par cellule) (nKat par cellules);
- une inhibition sensiblement totale de la flore indésirable se développant pendant le maltage ;
- une absence d'activité mutagène mesurée par le test d'Ames.

2. Souche selon la revendication 1 présentant en outre au moins une des caractéristiques suivantes :
- la stabilité de la solution de leurs spores est supérieure à 6 mois ;
- leur production de spores est supérieure à 10⁷ par ml.

3. Souche selon la revendication 2 **caractérisée en ce qu'**il s'agit des souches déposées à la CNCM sous les numéros 1.1474 et 1.1475 le 06 septembre 1994.

4. Procédé de maltage de céréales telles que blé, orge, riz, sorgho, maïs, sarrazin, **caractérisé en ce qu'**il comprend un ensemencement par une souche de Geotrichum candidum telle que définie dans l'une des revendications précédentes, dans des quantités de l'ordre de 10⁵ à 10⁹ Unités formant colonies (UFC) par kg de céréales.

5. Procédé selon la revendication 4 **caractérisé en ce que** l'ensemencement est effectué dans des quantités de 10⁷ à 10⁸ UFC par kg de céréales.

6. Procédé selon l'une des revendications 4 ou 5, **caractérisé par le fait que** l'ensemencement du micro-organisme est effectué en une seule ou en plusieurs fois au cours de l'une ou plusieurs des étapes suivantes : préparation des céréales ou autres végétaux, lors de la première trempe, de la dernière trempe, des trempes intermédiaires, au moment du transfert entre l'étape de trempe et l'étape de germination, lors de la germination, au moment du transfert entre l'étape de germination et l'étape de touraillage, ou lors du touraillage.

7. Procédé selon les revendications 4 à 6 **caractérisé en ce qu'**il s'agit des souches déposées sous les numéros 1-1474 ou 1-1475 auprès de la CNCM.

8. Utilisation d'un micro-organisme de la famille de Geotrichum candidum dans le maltage de céréales ou autres végétaux, tels que blé, orge, riz, sorgho, maïs, sarrasin, pour le contrôle du développement de la flore à même de contaminer et coloniser ces céréales ou autres végétaux.

9. Utilisation selon la revendication 8 **caractérisée par le fait que** le micro-organisme est composé d'une ou plusieurs souches sélectionnées, de Geotrichum candidum.

10. Utilisation selon la revendication 8 ou 9 **caractérisée en ce que** le micro-organisme est un Geotrichum candidum selon l'une des revendications 1 à 3.

11. Utilisation selon l'une quelconque des revendications 8 à 10, **caractérisée par le fait que** le micro-organisme est ensemencé en cours de maltage, soit sous forme de culture pure, soit sous forme de culture mixte en association avec un ou plusieurs autres micro-organismes.

12. Utilisation selon la revendication 11 **caractérisée par le fait que** le micro-organisme est ensemencé en alternance ou est associé sous forme de culture mixte avec des bactéries lactiques.

13. Utilisation selon l'une quelconque des revendications 8 à 12, **caractérisée par le fait que** l'ensemencement s'effectue dans des quantités de l'ordre de 10⁵ à 10⁹ de préférence 10⁷ à 10⁸ unités formant des colonies (UFC) par kilogramme de céréales ou autres végétaux.

14. Utilisation d'une souche selon l'une des revendications 1 à 3 dans le maltage de céréales ou autres végétaux, tels que blé, orge, riz, sorgho, maïs, sarrasin, pour le contrôle du phénomène de giclage de la boisson obtenue à partir du malt.

## Patentansprüche

1. Stämme von Mikroorganismen der Familie Geotrichum candidum, welche erhältlich sind durch Selektion und dann Klonen von Naturstämmen des Mikroorganismus und welche gleichzeitig aufweisen:
- eine Lipaseaktivität, die geringer ist als 2,5 x 10⁻⁷ Nanomole pro Sekunde pro Zelle (nKat pro Zellen);
- eine in etwa vollständige Inhibition der unerwünschten Flora, die sich während es Mälzens entwickelt;
- ein Fehlen einer mutagenen Aktivität, gemessen durch den Test von Ames.

2. Stamm nach Anspruch 1, welcher außerdem wenigstens eines der folgenden Kennzeichen aufweist:
- die Stabilität der Lösung seiner Sporen ist größer als 6 Monate;
- dessen Produktion von Sporen ist größer als 10⁷ pro ml.

3. Stamm nach Anspruch 2, **dadurch gekennzeichnet, dass** es sich um Stämme handelt, die bei der CNCM unter den Nummern I.1474 und I.1475 am 6. September 1994 hinterlegt wurden.

4. Verfahren zum Mälzen von Zerealien wie Weizen, Gerste, Reis, Sorghum, Mais, Buchweizen, **dadurch gekennzeichnet, dass** es ein Beimpfen mit einem Stamm von Geotrichum candidum, wie in einem der vorhergehenden Ansprüche definiert, bei Mengen in der Größenordnung von 10⁵ bis 10⁹ koloniebildenden Einheiten (UFC) pro kg Zerealien umfasst.

5. Verfahren nach Anspruch 4, **dadurch gekennzeichnet, dass** das Beimpfen bei Mengen von 10⁷ bis 10⁸ UFC pro kg Zerealien durchgeführt wird.

6. Verfahren nach einem der Ansprüche 4 oder 5, **gekennzeichnet durch** die Tatsache, dass das Beimpfen mit dem Mikroorganismus ein einziges oder mehrere Male während eines oder mehrerer der folgenden Schritte durchgeführt wird: Vorbereitung von Zerealien oder anderen Pflanzen, zur Zeit der ersten Maische, der letzten Maische, der dazwischenliegenden Maischen, im Moment der Überführung zwischen dem Schritt der Maische und dem Schritt der Keimung, zur Zeit der Keimung, im Moment der Überführung zwischen dem Schritt der Keimung und dem Schritt der Darre, oder zur Zeit der Darre.

7. Verfahren nach den Ansprüchen 4 bis 6, **dadurch gekennzeichnet, dass** es sich um Stämme handelt, die unter den Nummern I-1474 oder I-1475 bei der CNCM hinterlegt wurden.

8. Verwendung eines Mikroorganismus der Familie Geotrichum candidum beim Mälzen von Zerealien oder anderen Pflanzen wie Weizen, Gerste, Reis, Sorghum, Mais, Buchweizen zur Kontrolle der Entwicklung der Flora, welche im Stande ist, die Zerealien oder anderen Pflanzen zu kontaminieren und zu besiedeln.

9. Verwendung nach Anspruch 8, **gekennzeichnet durch** die Tatsache, dass der Mikroorganismus aus einem oder mehreren ausgewählten Stämmen von Geotrichum candidum zusammengesetzt ist.

10. Verwendung nach Anspruch 8 oder 9, **dadurch gekennzeichnet, dass** der Mikroorganismus ein Geotrichum candidum nach einem der Ansprüche 1 bis 3 ist.

11. Verwendung nach irgendeinem der Ansprüche 8 bis 10, **gekennzeichnet durch** die Tatsache, dass der Mikroorganismus während des Mälzens entweder in Form einer reinen Kultur oder in Form einer gemischten Kultur in Verbindung mit einem oder mehreren anderen Mikroorganismen beimpft wird.

12. Verwendung nach Anspruch 11, **gekennzeichnet durch** die Tatsache, dass der Mikroorganismus im Wechsel oder zusammen in Form einer gemischten Kultur mit Milchsäurebakterien beimpft wird.

13. Verwendung nach irgendeinem der Ansprüche 8 bis 12, **gekennzeichnet durch** die Tatsache, dass das Beimpfen bei Mengen in der Größenordnung von 10⁵ bis 10⁹, vorzugsweise 10⁷ bis 10⁸ koloniebildenden Einheiten (UFC) pro kg Zerealien oder anderer Pflanzen durchgeführt wird.

14. Verwendung eines Stammes nach einem der Ansprüche 1 bis 3 beim Mälzen von Zerealien oder anderen Pflanzen wie Weizen, Gerste, Reis, Sorghum, Mais, Buchweizen zur Kontrolle des Phänomens des Spritzens des Getränks, das ausgehend von dem Malz erhalten wird.

## Claims

1. Strains of micro-organisms from the Geotrichum candidum family that can be obtained by selection then cloning of natural strains of the micro-organism and simultaneously having:
• a lipase activity of less that 2.5 x 10⁻⁷ nanomoles per second per cell (nKat per cell);
• substantially complete inhibition of undesirable flora developing during malting;
• an absence of mutagenic activity, measured using the Ames test.

2. A strain according to claim 1, possessing at least one of the following further characteristics:
• a stability of more than 6 months for a solution of its spores;
• a spore production of more than 10⁷ per ml.

3. A strain according to claim 2, **characterized in that** it is a strain deposited on 6^{th} September 1994 at the CNCM with accession numbers I-1474 and I-1475.

4. A process for malting cereals such as wheat, barley, rice, sorghum, maize or buckwheat, **characterized in that** it comprises seeding with a strain of Geotrichum candidum as defined in any one of the preceding claims, in quantities of the order of 10⁵ to 10⁹ colony forming units (CFU) per kg of cereals.

5. A process according to claim 4, **characterized in that** seeding is carried out in quantities of 10⁷ to 10⁸ CFU per kg of cereals.

6. A process according to claim 4 or claim 5, **characterized in that** the micro-organism is seeded in one or in a plurality of passes during one or a plurality of the following steps: preparation of cereals or other plants, during the first mash, the last mash, intermediate mashes, at the time of transfer between the mash step and the germination step, during germination, at the time of transfer between the germination step and the kilning step, or during kilning.

7. A process according to claims 4 to 6, **characterized in that** the strains are those deposited at the CNCM with accession numbers I-1474 or I-1475.

8. Use of a micro-organism from the Geotrichum candidum family in malting cereals or other plants such as wheat, barley, rice, sorghum, maize or buckwheat, to control the development of flora capable of contaminating and colonising said cereals or other plants.

9. Use according to claim 8, **characterized in that** the micro-organism is composed of one or more selected strains of Geotrichum candidum.

10. Use according to claim 8 or claim 9, **characterized in that** the micro-organsim is a Geotrichum candidum according to any one of claims 1 to 3.

11. Use according to any one of claims 8 to 10, **characterized in that** the micro-organism is seeded during malting, either in the form of a pure culture, or in the form of a mixed culture in association with one or more other micro-organisms.

12. Use according to claim 11, **characterized in that** the microorganism is seeded in alternation, or is in association in the form of a mixed culture, with lactic bacteria.

13. Use according to any one of claims 8 to 12, **characterized in that** seeding is carried out in quantities of the order of 10⁵ to 10⁹, preferably 10⁷ to 10⁸ colony forming units (CFU) per kilogram of cereals or other plants.

14. Use of a strain according to any one of claims 1 to 3 in malting cereals or other plants, such as wheat, barley, rice, sorghum, maize or buckwheat, to control spirting of the beverage obtained from the malt.
